# EUROPÄISCHE PATENTSCHRIFT

(11) **EP 0 568 552 B1**
(45) Veröffentlichungstag und Bekanntmachung des Hinweises auf die Patenterteilung: **05.10.1994**
(21) Anmeldenummer: 92901853.9
(22) Anmeldetag: 23.12.1991
(51) Int. Cl.: A61F 2/36, A61F 2/32

(54) **ENDOPROTHESE DES HÜFTGELENKES**
ENDOPROSTHESIS FOR THE HIP JOINT
ENDOPROTHESE DE L'ARTICULATION DE LA HANCHE

(30) Priorität: 25.01.1991 AT 167/91
(43) Veröffentlichungstag der Anmeldung: 10.11.1993
(73) Patentinhaber: WINKLER, Heinz, A-1232 Wien (AT)
(72) Erfinder: WINKLER, Heinz, A-1232 Wien (AT)
(74) Vertreter: Brauneiss, Leo, Dipl.Ing.
(86) Internationale Anmeldenummer: AT9100139
(87) Internationale Veröffentlichungsnummer: WO9212691

(56) Entgegenhaltungen:
- EP-A- 0 091 315
- EP-A- 0 228 523
- WO-A-85/05027
- WO-A-86/03962
- US-A- 2 682 265
- US-A- 3 740 769

## Beschreibung

Die Erfindung betrifft eine Endoprothese des Hüftgelenkes gemäß den Merkmalen des Oberbegriffs des Anspruchs 1, wie sie aus der US-A-3 740 769 als bekannt hervorgeht. Endoprothesen werden dann implantiert, wenn durch gelenkerhaltende Operationen ein befriedigendes therapeutisches Ergebnis nicht mehr erzielbar ist. Bei einem Hüftgelenk ist dies insbesondere dann der Fall, wenn eine fortgeschrittene Coxarthrose, eine Hüftkopfnekrose oder eine mediale Fraktur des Schenkelhalses vorliegt.

Nachteil aller bekannten Endoprothesen ist es, daß diese zwar zunächst mit den Knochen unverrückbar verbunden sind, sich jedoch nach einiger Zeit lockern können. Lebender Knochen nämlich ist jedoch stetigen Veränderungen ausgesetzt, welche von Lokalisation zu Lokalisation äußerst unterschiedlich sein können. Kommt es im Zuge solcher Veränderungen zu einer Schwächung einzelner Abschnitte des Knochens im Prothesenrandbereich, so wird der Knochen an dieser Stelle nachgeben und die Prothese an dieser Stelle nachsetzen. Auch wenn sich solche Bewegungen nur im Mikrobereich abspielen, so ist dadurch die ursprünglich gegebene Stabilität nicht mehr gewährleistet. Bei instabilen Verhältnissen kommt es zu ständig wechselnden Kraftflüssen und Belastungsverteilungen am Knochen. Dieser kann daher keine reparativen Reaktionen setzen, da die Voraussetzungen für ein gerichtetes Wachstum fehlen. Er wird vielmehr an Stellen mit überhöhter Belastung (stress reaction) ebenso mit isoliertem Abbau reagieren wie an Stellen mit zu geringer Belastung (stress protection). Dadurch kommt es zu einem Lockerungsprozeß, der progredient fortschreitet.

Mit derartigen Umbauvorgängen im Knochen muß auch noch Jahre nach abgeschlossener Einheilung der Prothese gerechnet werden, da der Knochen als lebendes Organ auf geänderte Lebens-, Ernährungs- und sonstige Umstände reagiert. Bei herkömmlichen Endoprothesen wird dieser Umstand nicht berücksichtigt.

Ein solcher Lockerungsprozeß ist eines der größten Probleme in der orthopädischen Chirurgie und erfordert häufig einen Prothesenwechsel. Die hiefür notwendigen Revisionsoperationen sind jedoch ungleich problematischer als Primäreingriffe, da nicht nur das bei der Primärimplantation entfernte Knochengewebe fehlt, sondern zumeist durch das gelockerte Implantat bzw. den entstandenen Abrieb zusätzlich schwere Defekte entstehen, welche eine neuerliche Implantatfixierung massiv erschweren oder sogar verhindern.

Bei Hüftendoprothesen wird fallweise lediglich der Femurkopf und -hals entfernt und durch eine Kopfprothese ersetzt.

Bei den bekannten Kopfprothesen besteht der Verankerungsteil in der Regel aus einem Spieß aus Metall, beispielsweise aus Titan, der im Femurmarkraum fixiert wird und mit einem seitlich abstehenden Zapfen versehen ist, dessen freies Ende einen Kopfteil trägt. Zu dessen Implatation ist es erforderlich, den Femurkopf und den Femurhals sowie weitgehend die spongiösen Knochenanteile im Bereich des proximalen Oberschenkels zu entfernen. Der Spieß wird hierauf im Femurmarkraum mit Polymethylmethacrylat einzementiert oder zementfrei eingeschlagen, wobei im letzteren Fall primär ein optimaler Kontakt zwischen dem Knochen und dem Implantat gefordert wird, welcher gegebenenfalls durch sekundären Einwuchs neuen Knochengewebes gefestigt werden kann.

Aus der DE-A 28 45 231 ist eine Gelenkprothese bekannt geworden, bei der ein Gelenkteil mit einem Schaft versehen ist, der mit Hilfe einer Zugschraube mit dem Knochen verspannt wird.

Aus der DE-A 28 54 334 ist eine kompliziert aufgebaute Endoprothese für ein Hüftgelenk bekannt geworden, die aus einem in Längsrichtung des Knochens verlaufenden Markraumschaft und einem in Richtung des Schenkelhalses verlaufenden, den Gelenkkopf tragenden Schenkelhalsteil besteht. Der Markraumschaft ist hiebei mit dem Schenkelhalsteil starr verbunden.

Die DE-A 30 17 953 offenbart eine Endoprothese für einen Femurkopf, bei welchem der Kopfteil über einen Schraubenbolzen starr und unverschiebbar mit einem Verankerungsteil verbunden ist, der aus einer in den Knochen eingesetzten Hülse und aus einer an der Außenseite des Knochens angeschraubten Trochanterplatte besteht. Der Schraubenbolzen wirkt hiebei als Zuganker, durch welchen die Prothese am Knochen unter Vorspannung gehalten wird.

Die DE-A 34 20 035 beschreibt eine Gelenkprothese, bei welcher der Gelenkkopf über einen Fortsatz starr mit dem Knochen verspannt ist.

Allen diesen bekannten Gelenkprothesen ist gemeinsam, daß eine starre, unnachgiebige Verbindung mit dem Knochen erfolgt, welche späteren Umbauvorgängen im Knochen nicht Rechnung trägt.

Beim Implantieren einer Pfannenprothese wird im Bereich des Beckens teilweise auch intaktes Knochenmaterial entfernt, um die Pfanne in gleicher Weise einzementieren bzw. zementfrei verankern zu können. Diese bekannte Operationsmethode unter Verwendung bekannter Endoprothesen weist eine Reihe von Nachteilen auf. So müssen große Mengen von intaktem Knochenmaterial geopfert werden, darunter gerade jene Knochenanteile im Bereich des proximalen Femurs, welche durch ihre sinnvolle trajektorielle Anordnung eine optimale Krafteinleitung bewirken.

Im Femurschaftbereich wird eine Umorientierung der bestehenden Kraftflüsse bewirkt. Während unter natürlichen Verhältnissen in diesem Bereich vor allem reine Biegebeanspruchungen mit einer harmonischen Verteilung von oben nach unten vorherrschen, entstehen nach Prothesenimplantation Kräfte, welche hauptsächlich von innen nach außen verlaufen, kombiniert mit Scherkräften und einem relativ abrupten Übergang von geringer Belastung in den der Prothese anliegenden Knochenanteilen zu extremer Belastung im Bereich der unteren Spitze des Spießes. Der Eigenknochen muß mit Umbauvorgängen auf die geänderten Bedingungen reagieren. Dies ist sowohl für die femoralen als auch die acetabulären Verhältnisse zutreffend. Umbau bedeutet stets gleichzeitig Ab- und Anbau von Knochen. Wenn nun der Abbau den Anbau mengenmäßig übersteigt, verliert die Prothese in einzelnen Abschnitten ihren Halt und beginnt sich zu lockern.

Auch auf die individuell verschiedenen Verhältnisse in der Anatomie des Patienten wird bei der Verwendung bekannter Hüftgelenkendoprothesen nur ungenügend eingegangen. So weist der Winkel zwischen Oberschenkelschaft und Schenkelhals ("CCD-Winkel") von Mensch zu Mensch unterschiedliche Größen auf (115 bis 140 Grad gelten als physiologisch). Weiters verläuft der Schenkelhals nicht in der Sagittalebene des Körpers, sondern ist in einem unterschiedlich großen Winkel gegen diese nach vorne gerichtet ("AT-Winkel, physiologisch ca. 10 bis 40 Grad). Beide Winkel sind voneinander in gesetzmäßiger Weise abhängig. Bekannte Endoprothesen nehmen darauf keine Rücksicht, es gibt für alle Hüften nur ein Standardmaß. Daraus ergibt sich fast immer eine funktionelle Gelenksfehlstellung und dadurch zusätzlich eine veränderte Krafteinleitung in den Oberschenkelschaft. Knochen wie Weichteile müssen sich, sofern sie dazu in der Lage sind, den geänderten Verhältnissen anpassen. Schmerzen, Einschränkungen der Beweglichkeit und vorzeitige Prothesenlockerungen sind die mögliche Folge. Individuell angefertigte Prothesen sind nicht sofort verfügbar, zumeist unpräzis und mit einem bis zum zehnfachen finanziellen Aufwand verbunden. Weiters ist zur präoperativen Bestimmung der vorliegenden anatomischen Verhältnisse eine Computertomographie mit hoher Strahlenbelastung des Patienten notwendig. Auch durch diese funktionelle Gelenksfehlstellung ergibt sich die Gefahr einer Lockerung der implantierten Prothese mit den bereits erwähnten Nachteilen.

Besiedelung der Prothesenoberfläche mit Bakterien ist eine nicht seltene und äußerst gefürchtete Komplikation nach deren Implantation. Eine solche macht zumeist die vollständige Entfernung der Prothese notwendig. Die Entfernung einer fest eingewachsenen Endoprothese bzw. des Knochenzements ist jedoch mit großen Schwierigkeiten und häufig enormen zusätzlichen Schäden an den umliegenden Knochenstrukturen verbunden. Eine neuerliche Prothesenversorgung ist mitunter gar nicht oder erst nach jahrelanger Ausheilungszeit durchführbar. Besteht eine Infektion im Bereich einer bekannten Kopfprothese, so muß wegen ihrer Verankerung im Femurmarkraum mit einer Ausbreitung der Infektion bis in den Bereich des Kniegelenks gerechnet werden.

Die Verankerung der Kopfprothese im Schaftbereich ist nicht nur unphysiologisch, sondern auch technisch nicht immer einfach. Ein umfangreiches Zusatzinstrumentarium ist zumeist Voraussetzung für eine exakte Präparation des Knochens. Weiters hat die Eröffnung des Markraumes stets heftige und lang anhaltende Blutungen zur Folge, welche immer die mehrfache Gabe von Blutkonserven notwendig macht.

Die vorliegende Erfindung hat sich zur Aufgabe gestellt, dieerwähnten Nachteile zu vermeiden und eine Endoprothese zu schaffen, welche sich insbesondere später möglichen Veränderungen des Knochens in kontrollierter Weise anzupassen vermag. Zur Lösung dieser Aufgabe schlägt die Erfindung ausgehend von einer Endoprothese der eingangs beschriebenen Art, die Kennzeichnenden Merkmale des Anspruchs 1 vor. Durch die erfindungsgemäße Ausbildung der Endoprothese ist somit eine geführte Verschiebbarkeit des Glenkteiles relativ zum Verankerungsteil gewährleistet und zwar in einer genau vorbestimmten axialen Richtung, nämlich in Richtung des physiologischen Kraftflusses, während des gesamten Zeitraumes, in dem die Endoprothese in Funktion ist. Dadurch werden im wesentlichen zwei Vorteile erzielt. Einerseits ermöglicht diese geführte Verschiebbarkeit ein axiales Nachsetzen im Falle von lokalisiertem Knochenabbau, sodaß die Stabilität der Prothese über einen praktisch unbegrenzten Zeitraum nach der Implantation erhalten bleibt, wogegen bei den bekannten Prothesen die Gefahr besteht, daß sich infolge von Umbauvorgängen im lebenden Knochen nach abgeschlossener Einheilung der Prothese diese lockert bzw. nicht mehr ihre optimale Lage einnimmt, andererseits wird dadurch gewährleistet, daß sich die erfindungsgemäße Prothese selbsttätig in optimaler Weise einstellt und dadurch eine optimale Belastungsverteilung zwischen der Prothese und dem Knochen sichergestellt ist. Im Verankerungsteil findet bei der erfindungsgemäßen Ausbildung nur eine untergeordnete Übernahme von Kraftflüssen statt. Diese Kraftflüsse erfolgen vielmehr wie beim natürlichen Gelenk durch die präformierten Knochenstrukturen.

Die erfindungsgemäße Kopfprothese mit einem am Femur verankerbaren Verankerungsteil, der über einen Halsteil mit einem darauf befestigten Kopfteil in Verbindung steht, ist dadurch gekennzeichnet, daß der Halsteil mit dem stabförmigen Gleitteil verbunden ist, der in einer mit dem Verankerungsteil verbundenen rohrförmigen Führung im wesentlichen in Richtung der Schenkelhalsachse verschiebbar angeordnet ist, und der mit einem am Schenkelhals anliegenden Ansatz versehen ist. Dadurch wird sichergestellt, daß ein physiologischer Kraftfluß erfolgt, sodaß im Femur auch nach Implantieren der Prothese natürliche Verhältnisse hinsichtlich der Kraftverteilung herrschen und nicht einzelne Knochenanteile unphysiologisch belastet werden und auf diese Belastung mit Umbauvorgängen reagieren.

Gleichzeitig wird über den Ansatz eine sichere Verankerung der Prothese am Femur erzielt, ohne daß größere Mengen von intaktem Knochenmaterial entfernt werden müssen.

Gemäß einer bevorzugten Ausführungsform der Erfindung ist hiebei die am Schenkelhals anliegende Fläche des Ansatzes kegelstumpfförmig ausgebildet, wobei die Spitze des Kegels nach lateral distal gerichtet ist und die Achse des Kegels mit der Achse des stabförmigen Gleitteiles zusammenfällt. Der Spitzenwinkel des Kegels beträgt hiebei zweckmäßig zwischen 135° und 140°. Dadurch wird sichergestellt, daß die Kontaktflächen des Ansatzes mit dem Knochen in allen Anteilen im wesentlichen senkrecht auf die präformierten trabekulären Strukturen stehen. Weiters ist dadurch gewährleistet, daß die anatomisch vorgegebenen Verhältnisse zwischen CCD- und AT-Winkel nach der Prothesenversorgung gewahrt bleiben. Die Kraftübertragung erfolgt daher in weitgehend physiologischer Weise über die natürlich vorgegebenen Strukturen.

Gemäß einem weiteren Merkmal der Erfindung weist der Halsteil einen zentral des kegelstumpfförmig ausgebildeten Ansatzes angeordneten Konus auf, dessen Achse im wesentlichen mit der Schenkelhalsachse zusammenfällt und der in eine konusförmige Ausnehmung des Kopfteiles eingesetzt ist. Durch diese Ausführung wird es möglich, Kopfteile von bisher bekannten Prothesen bei der erfindungsgemäßen Endoprothese zu verwenden, diese somit mit bekannten Kopfteilen zu kombinieren.

Der Verankerungsteil weist gemäß einer bevorzugten Ausführungsform der Erfindung eine bei dynamischen Hüftschrauben an sich bekannte, am Femur laterial anschraubbare Platte auf, die mit der den Schenkelhals im wesentlichen in Richtung der Schenkelhalsachse durchsetzenden rohrförmigen Führung verbunden ist, in der der Gleitteil verschiebbar geführt ist. Bei dieser Anordnung ist es nicht erforderlich, größere Anteile noch intakten Knochenmaterials zu entfernen, die bei den bisher bekannten Endoprothesen geopferten Anteile des Knochens werden somit weitgehend geschont. Es ist lediglich notwendig, ein Bohrloch in Richtung der Schenkelhalsachse anzuordnen, durch das die Hülse hindurchgeführt werden kann. Durch das laterale Anschrauben der Platte erfolgt eine Fixierung des Verankerungsteiles, wobei sich eine langwierige, ein aufwendiges Instrumentarium erfordernde Präparation des Femurs erübrigt. Dadurch, daß der Verankerungsteil nicht im Femurmarkraum verankert wird, werden auch lang anhaltende Blutungen aus dem Markraum vermieden, wodurch die Gabe von Blutkonserven wesentlich reduziert, wenn nicht sogar unnötig ist. Im Falle einer Keimbesiedelung ist der Verankerungsteil leicht zu entfernen und ein Infektionsprozeß kann sich nicht über den gesamten Oberschenkel ausbreiten, weshalb eine sofortige Neuimplantation einer anderen Prothese mit einem wesentlich geringeren Risiko behaftet ist. Diese Ausführung weist weiters den Vorteil auf, daß bei einem allfälligen Versagen der erfindungsgemäßen Endoprothese der Ersatz derselben durch eine bekannte Prothese problemlos wie bei einer Primäroperation durchgeführt werden kann, da bei der Implantation der erfindungsgemäßen Endoprothese an der erforderlichen Knochensubstanz keinerlei Defekte gesetzt wurden.

Die erfindungsgemäße Pfannenprothese ist, ausgehend von einer bekannten, in einer Ausfräsung des Beckenknochens verankerten Pfanne, die an ihrem cranialen Rand mit wenigstens einer Lasche versehen ist, die über Befestigungsmittel mit dem Beckenknochen verbindbar ist, dadurch gekennzeichnet, daß wenigstens zwei mit der bzw. den Lasche(n) verbundene rohrförmige Führungen vorgesehen sind, deren Achsen senkrecht zur Pfanneneingangsebene verlaufen, und die von am Beckenknochen verankerbaren, stabförmige Gleitteile bildenden bzw. aufweisenden Schrauben durchsetzt sind, die relativ zu den rohrförmigen Führungen verschiebbar angeordnet sind. Auch diese Ausführungsform stellt sicher, daß bei einer Veränderung der Knochenstruktur infolge der Verschiebbarkeit ein axiales Nachsetzen der Pfanne erfolgen kann, diese jedoch weder kippen noch rotieren kann, so daß auch in Fällen von lokalisiertem Knochenabbau die Stabilität der Prothese jederzeit gewahrt bleibt, ohne daß der Kraftfluß zwischen der Prothese und dem Knochen gestört oder unterbrochen wird. Auch bei einem etwaigen Setzungsvorgang bleiben somit sämtliche Anteile der Pfannenzirkumferenz einer gleichmäßigen Belastungsverteilung ausgesetzt. Im Gegensatz dazu besteht bei den bekannten Pfannenprothesen die Gefahr eines Kippens, wodurch der Kraftfluß gestört wird. Weiters ermöglicht diese Ausbildung nach dem Setzen der Schrauben eine Verschiebung der Pfanne senkrecht zur Pfanneneingangsebene, sichert jedoch gleichzeitig die Pfanne gegen eine Kipp- und Rotationsbewegung.

Damit durch das Gewinde der Schrauben die Verschiebbarkeit in Richtung der Schraubenachse nicht behindert wird, weisen gemäß einem weiteren Merkmal der Erfindung die Schrauben einen zentralen, mit einem Gewinde versehenen Verankerungsabschnitt und einen peripheren, in der zugehörigen Gleithülse verschiebbar geführten Gleitteil auf.

Bei der Präparation des knöchernen Lagers für die erfindungsgemäße Pfannenprothese ist es nicht mehr notwendig, wie bisher sämtliche sklerosierten Knochenanteile zu entfernen oder gar zusätzliche Defekte wie eine konische oder mit einem Schraubengewinde versehene Präparation zu setzen, es genügt vielmehr eine kongruente halbkugelförmige Präparation mit herkömmlichen Raspeln bis zur Entfernung noch vorhandener Knorpelreste, da Inhomogenitäten der Knochenstruktur dank der autoregulativen Eigenschaft der erfindungsgemäßen Pfannenprothese ausgeglichen werden können. Auch hier wird somit die Opferung von an sich intaktem Knochenmaterial auf ein absolutes Minimum reduziert.

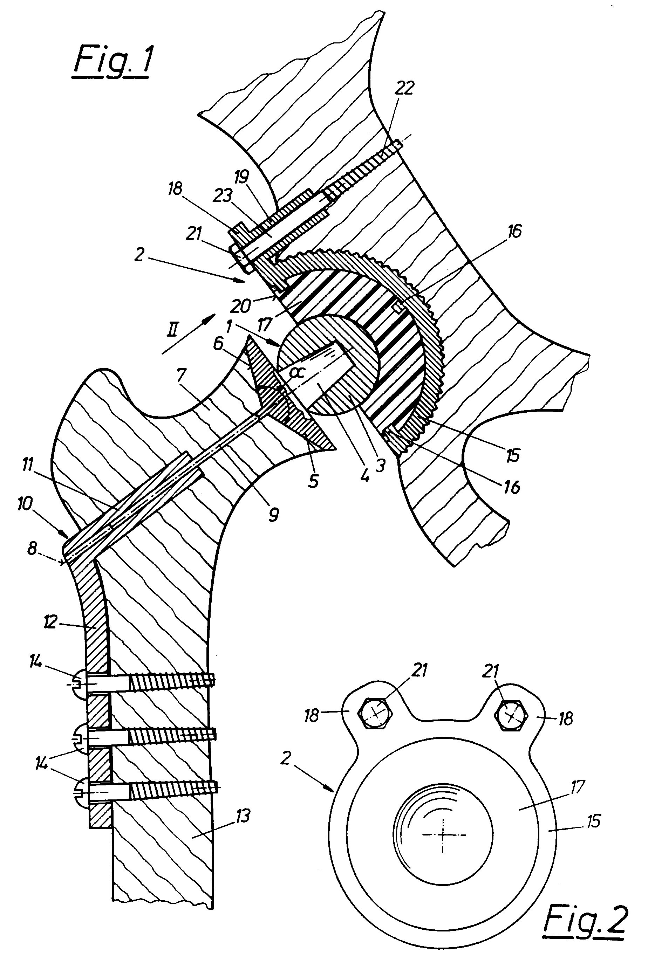

In der Zeichnung ist die Erfindung anhand eines Ausführungsbeispieles schematisch dargestellt. Fig.1 zeigt eine Totalendoprothese des Hüftgelenkes. Fig.2 stellt eine Ansicht der Pfannenprothese in Richtung des Pfeiles II in Fig.1 dar.

Die dargestellte Totalhüftendoprothese weist eine Kopfprothese 1 und eine Pfannenprothese 2 auf.

Die Kopfprothese 1 besteht aus einem Kopfteil 3, der auf einen Konus 4 aufgesetzt ist. Der Konus 4 ist mit einem Ansatz 5 verbunden, der eine kegelstumpfförmig ausgebildete Fläche 6 aufweist, die an einer durch Ausfräsen hergestellten Ausnehmung des Schenkelhalses 7 anliegt. Die Spitze des Kegels ist somit nach lateral distal gerichtet, die Achse des Kegels fällt im wesentlichen mit der Achse 8 des Schenkelhalses 7 zusammen.

Der Spitzenwinkel α des Kegels beträgt etwa 135° bis 140°, sodaß die Fläche 6 des Ansatzes 5 etwa senkrecht auf die präformierten trabekulären Strukturen steht.

Der Konus 4 und der Ansatz 5 bilden zusammen einen Halsteil, der mit einem von der Spitze des Kegels ausgehenden stabförmigen Gleitteil 9 verbunden ist, der in einem Verankerungsteil 10 in Richtung der Schenkelhalsachse 8 verschiebbar geführt ist. Hiezu weist der Verankerungsteil 10 eine Hülse 11 auf, die in eine in Richtung der Schenkelhalsachse 8 verlaufende, nach lateral distal offene Bohrung des Schenkelhalses 7 eingesetzt ist. Die Hülse 11 ist mit einer Platte 12 verbunden, die mit Löchern versehen ist, durch welche im Femur 13 eingeschraubte Schrauben 14 hindurchgeführt sind.

Die Pfannenprothese 2 besteht aus einer kugelkalottenförmigen Pfanne 15 aus einem Metall hoher Biokompatibilität, beispielsweise aus Titan, die mit üblichen Haltevorrichtungen 16 zum stabilen Einsetzen eines Inlays 17 aus gleitfähigem Material, beispielsweise aus Polyethylen, versehen ist. Wie aus Fig.2 hervorgeht, ist die Pfanne 15 an ihrem cranialen Rand mit zwei Laschen 18 verbunden, von welchen jede eine Gleithülse 19 aufweist, wobei die Achsen der Gleithülsen senkrecht auf die Pfanneneingangsebene 20 verlaufen. Es kann aber jede Lasche 18 auch zwei oder mehr von den Schrauben 21 durchsetzte Gleithülsen 19 aufweisen und es ist auch möglich, lediglich eine einzige Lasche mit zumindest zwei Gleithülsen 19 vorzusehen. Nach Implantation der Pfanne 15 wird diese durch die Gleithülsen 19 durchsetzende Schrauben 21 im Acetabulum fixiert. Die Schrauben 21 weisen einen zentralen, mit einem Gewinde versehenen Verankerungsabschnitt 22 und einen peripheren, in den Gleithülsen 19 verschiebbar geführten Gleitabschnitt 23 auf.

Diese Anordnung gewährleistet eine Fixierung der Pfanne 15 gegen Kippen und Verdrehen, ermöglicht jedoch dadurch, daß die Schraubenachsen senkrecht auf die Pfanneneingangsebene 20 stehen, ein axiales Nachsetzen im Falle von lokalisiertem Knochenabbau, wobei die Stabilität erhalten bleibt.

Beim Implantieren der in der Zeichnung dargestellten Totalendoprothese wird wie folgt vorgegangen:

Nach Eröffnung der Gelenkskapsel und nach Luxation des Hüftkopfes wird dieser in Höhe der Knorpel-Knochengrenze reseziert, wobei die Resektionsebene möglichst senkrecht auf die Schenkelhalsachse 8 liegen soll. Im Falle einer medialen Schenkelhalsfraktur werden nach Entfernung des abgebrochenen Kopfes lediglich stärker vorspringende corticale Spitzen im Bereich des Halsstumpfes geglättet. Hierauf wird von der Resektionsfläche ausgehend ein zentraler Bohrdraht in Richtung der Schenkelhalsachse 8 vorgelegt, welcher den gesamten Schenkelhals 7 durchsetzt und lateral aus dem Femur 13 austritt. Die zentrale Lage des Drahtes kann mittels eines Zielgerätes oder mittels Röntgenkontrolle (Bildwandler) sichergestellt werden. In der Folge wird von lateral über den liegenden Bohrdraht mit einem Mehrstufenbohrer aufgebohrt, so daß ein Bohrloch größeren Durchmessers für die Aufnahme der Hülse 11 des Verankerungsteiles 10 und ein Bohrloch kleineren Durchmessers für die Aufnahme des Gleitteils 9 gebildet wird. Der Winkel zwischen Bohrloch und Femurschaft wird gemessen. Diesem Winkel entsprechend wird der Verankerungsteil 10 gewählt, eingesetzt und mittels der Schrauben 14 am Femur 13 fixiert. Anschließend wird von medialseitig die Präparation der Aufnahmefläche für den Ansatz 5 durchgeführt. Diese erfolgt mittels einer kegelstumpfförmigen Raspel, deren Form exakt der des Ansatzes 5 entspricht und welche an ihrer Spitze mit einem Führungsstab versehen ist, welcher in die bereits eingesetzte Hülse 11 eingeführt wird. Die Präparation erfolgt, bis eine Glättung aller corticalen Ränder des Schenkelhalsstumpfes erzielt ist.

Es folgt die Präparation des Pfannenbettes mittels einer herkömmlichen halbkugelförmigen Raspel, deren Durchmesser dem größten Durchmesser des Acetabulums entsprechen sollte. In das geschaffene Bett wird eine Probepfanne gleicher Form eingesetzt, welche an ihrem cranialen Rand mit mindestens zwei senkrecht auf die Pfanneneingangsebene stehenden Führungsbuchsen zur Aufnahme eines Bohrers versehen ist. Nach Ermittlung der optimalen Lage der zu implantierenden Pfannenprothese durch Kippen bzw. Drehen der Probeprothese werden durch die Führungsbuchsen die Bohrlöcher zur Aufnahme der Schrauben 21 gesetzt. Die Probeprothese wird entfernt, die Länge der Bohrlöcher wird gemessen. Mittels eines Versenkungsbohrers konstanter Bohrtiefe werden die Bohrlöcher in ihrem peripheren Anteil zur Aufnahme der Gleithülsen 19 der Pfannenprothese 2 erweitert. Sodann wird die Original-Pfannenprothese 2 mit entsprechendem Durchmesser derart eingesetzt, daß deren Gleithülsen 19 in die dafür angelegten Ausnehmungen zu liegen kommen. Die Prothese wird fest angeschlagen und anschließend mittels der Schrauben 21 entsprechender Länge durch die Gleithülsen 19 fixiert. Der Gleitteil 23 der Schrauben 21 muß dabei stets das zentrale Ende der Gleithülse 19 überragen.

Es wird sodann die mittlerweile vorbereitete Kopfprothese 1 von medialseitig her mit dem Gleitteil 9 voran in das für sie geschaffene Knochenlager eingeführt und fest angeschlagen. Das periphere Ende des Gleitteils 9 sollte sodann etwa mit der lateralen Austrittsöffnung der Hülse 11 abschließen. Nach Aufschlagen des Kopfteils 3 auf den Konus 4 wird der Kopfteil 3 in die Pfannenprothese 2 reponiert, die Implantation ist abgeschlossen.

Obwohl in der Zeichnung eine Hüftendoprothese dargestellt ist, kann das Prinzip der Erfindung mit den gleichen Vorteilen auch für die prothetische Versorgung anderer Gelenke, insbesondere des Kniegelenkes, Anwendung finden. Wesentlich für alle Prothesentypen ist die geführte Verschiebbarkeit eines Gelenkteils gegen einen Verankerungsteil in axialer Richtung, sodaß im Falle lokalisierten Knochenabbaus eine Sicherung der Prothesenteile gegen Verkippen oder Verdrehen besteht, ohne daß der Kraftfluß über natürlich vorgegebene Knochenstrukturen unterbrochen wird.

Im Kniegelenksbereich wird der Verankerungsteil in Form je einer Gleithülse in den Femur- bzw. Tibiaschaft implantiert. In diesem ist ein vorzugsweise stabförmiger Gleitteil gleitend geführt, welcher mit dem femoralen bzw. tibialen Gelenksteil beliebigen Designs fest verbunden ist.

Im Bereich der Schulter wird der humerale Verankerungsteil analog dem Vorgehen am Hüftgelenk an der Lateralseite des Humerus befestigt. In diesem ist wiederum ein vorzugsweise stabförmiger Gleitteil gleitend geführt, welcher mit einem kugelkalottenförmigen Gelenksteil fest verbunden ist. Der glenoidale Prothesenanteil besteht zweckmäßigerweise aus einem hülsenförmigen Verankerungsteil, welcher im Schulterblatt befestigt wird und in dem ein vorzugsweise stabförmiger Gleitteil gleitend geführt ist, welcher mit einem schalenförmigen Gelenksteil fest verbunden ist.

## Patentansprüche

1. Endoprothese des Hüftgelenkes, mit einem am Knochen verankerbaren Verankerungsteil (21), der mit einem Gelenkteil (15) in Verbindung steht, wobei einer dieser beiden Teile (15) eine rohrförmige Führung (19) und der andere dieser beiden Teile (21) einen stabförmigen Gleitteil (23) aufweist, der in der rohrförmigen Führung (19) in Richtung des physiologischen Kraftflusses verschiebbar angeordnet ist, dadurch gekennzeichnet, daß die Endoprothese eine in einer Ausfräsung des Beckenknochens verankerbare kugelkalottenförmige Pfanne (15) aufweist, die an ihrem cranialen Rand mit wenigstens einer Lasche (18) versehen ist, die über Befestigungsmittel mit dem Beckenknochen verbindbar ist, wobei wenigstens zwei mit der bzw. den Lasche(n) (18) verbundene, sich in dieselbe Richtung wie die Pfanne (15) erstreckende rohrförmige Führungen (19) vorgesehen sind, deren Achsen senkrecht zur Pfanneneingangsebene verlaufen, und die von am Beckenknochen verankerbaren, stabförmige Gleitteile (23) bildenden bzw. aufweisenden Knochenschrauben (21) durchsetzt sind, die relativ zu den rohrförmigen Führungen (19) verschiebbar angeordnet sind.

2. Endoprothese nach Anspruch 1, dadurch gekennzeichnet, daß die Schrauben (21) einen zentralen, mit einem Gewinde versehenen Verankerungsabschnitt (22) und einen peripheren, in der zugehörigen Gleithulse (19) verschiebbar geführten Gleitteil (23) aufweisen.

3. Endoprothese nach Anspruch 1, bei welcher in der Pfanne (15) ein Kopfteil (3) gelenkig gelagert ist, der über einen Halsteil (4,5) mit einem am Femur (13) verankerbaren Verankerungsteil (10) in Verbindung steht, wobei der Halsteil (4, 5) mit dem stabförmigen Gleitteil (9) verbunden ist, der in einer mit dem Verankerungsteil (10) verbundenen rohrförmigen Führung (11) im wesentlichen in Richtung der Schenkelhalsachse (8) verschiebbar angeordnet ist, und der mit einem am Schenkelhals (7) anliegenden Ansatz (5) versehen ist, dadurch gekennzeichnet, daß die am Schenkelhals anliegende Fläche (6) des Ansatzes (5) kegelstumpfförmig ausgebildet ist, wobei die Spitze des Kegels nach lateral distal gerichtet ist und die Achse des Kegels mit der Achse des stabförmigen Gleitteiles (9) zusammenfällt.

4. Endoprothese mach Anspruch 3, dadurch gekennzeichnet, daß der Spitzenwinkel (α) des Kegels zwischen 135° und 140° beträgt.

5. Endoprothese nach Anspruch 3 oder 4, dadurch gekennzeichnet, daß der Halsteil (4,5) einen zentral des kegelstumpfförmig ausgebildeten Ansatzes (5) angeordneten Konus (4) aufweist, dessen Achse im wesentlichen mit der Schenkelhalsachse zusammenfällt und der in eine konusförmige Ausnehmung des Kopfteiles (3) eingesetzt ist.

## Claims

1. An endoprosthesis for a hip joint, having an anchoring component (21) fixed to the bone and connected with a joint component (15), in which one of these two components (15) possesses a tubular guide (19) and the other of these components (21) possesses a rod-shaped sliding component (23) which is shiftably arranged in the tubular guide (19) along the path of the physiological force flow, characterized in that the endoprosthesis contains a hemispherical socket (15) anchored in a recess cut in the pelvic bone, said socket being provided at its cranial margin with at least one bracket (18) which is connectible to the pelvic bone by means of fastening elements, whereby at least two tubular guides (19) are provided which are connected with the bracket(s) (18) and extend in the same direction as the socket (15) with their axes running perpendicular to the plane of the socket opening, and through these guides pass screws (21) forming or possessing rod-shaped sliding sections (23), said screws being anchored in the pelvic bone and slidingly arranged relative to the tubular guides (19).

2. An endoprosthesis according to Claim 1, characterized in that the screws (21) possess a central threaded anchoring section (22) and a peripheral sliding section (23) slidingly mounted in the associated sliding sleeve (19).

3. An endoprosthesis according to Claim 1, in which a head component (3) is articulatedly mounted in the socket (15), said head component being joined via a neck component (4,5) with an anchoring component (10) connected to the femur (13), and the neck component (4,5) is connected to the rod-shaped sliding component (9) which is slidingly arranged substantially along the axis of the femoral neck (8) in a tubular guide (11) joined to the anchoring component (10), said sliding component having an attachment (5) bearing on the neck of the femur (7), characterized in that the surface (6) of the attachment (5) has the form of a truncated cone, the vertex of which is laterally and distally oriented and the axis of which coincides with the axis of the rod-shaped sliding component (9).

4. An endoprosthesis according to Claim 3, characterized in that the vertex angle (α) of the truncated cone is between 135° and 140°.

5. An endoprosthesis according to Claim 3 or 4, characterized in that the neck component (4,5) possesses a cone (4) arranged centrally to the truncated conical attachment (5), and the axis of this cone (4) coincides substantially with the axis of the femoral neck, and the said cone is inserted into a comical recess in the head component (3).

## Revendications

1. Endoprothèse de l'articulation de la hanche, comportant un élément d'ancrage (21) ancrable à l'os, relié à un élément d'articulation (15), l'un (15) de ces deux éléments présentant un guide tubulaire (19) et l'autre (21) de ces deux éléments présentant un élément coulissant (23) en forme de barre, agencé dans le guide tubulaire (19) de manière à pouvoir se déplacer dans la direction physiologique des forces, caractérisée en ce que l'endoprothèse présente une cupule (15) en forme de calotte sphérique, susceptible d'être ancrée dans un logement fraisé dans l'os du bassin, laquelle cupule est dotée, sur son bord antérieur, d'au moins une attache (18) susceptible d'être reliée au moyen d'éléments de fixation à l'os du bassin, ladite endoprothèse comprenant au moins deux guides tubulaires (19) reliés à la ou aux attache(s) (18), orientés dans la même direction que la cupule (15) et dont les axes sont perpendiculaires au plan de la cupule, lesdits guides étant traversés par des vis à os (21) formant ou comportant des éléments coulissants (23) en forme de barre, ancrables au niveau de l'os de bassin, lesdites vis étant mobiles par rapport aux guides tubulaires (19).

2. Endoprothèse selon la revendication 1, caractérisée en ce que les vis (21) présentent une portion d'ancrage (22) centrale, dotée d'un filetage, et un élément coulissant (23) périphérique, guidé mobile dans la douille coulissante (19) correspondante.

3. Endoprothèse selon la revendication 1, dans laquelle une tête (3) reliée par un col (4, 5) à un élément d'ancrage (10) ancrable au fémur (13) est articulée dans la cupule (15), le col (4, 5) étant relié à l'élément coulissant (9) en forme de barre, mobile dans un guide tubulaire (11) relié à l'élément d'ancrage (10), essentiellement dans la direction de l'axe du col du fémur (8), et étant doté d'un embout (5) reposant contre le col du fémur (7), caractérisé en ce que la surface (6) de l'embout (5) reposant contre le col du fémur est de forme tronconique, la pointe du cône ayant une orientation latérale distale et l'axe du cône coïncidant avec l'axe de l'élément coulissant (9) en forme de barre.

4. Endoprothèse selon la revendication 3, caractérisée en ce que l'angle au sommet (α) du cône est compris entre 135° et 140°.

5. Endoprothèse selon la revendication 3 ou 4, caractérisée en ce que le col (4, 5) présente un cône (4) centré par rapport à l'embout tronconique (5), dont l'axe coïncide essentiellement avec l'axe du col du fémur et qui est inséré dans un évidement conique de la tête (3).
